(19) Europäisches Patentamt
European Patent Office
Office européen des brevets

(11) Veröffentlichungsnummer: **0 136 499**
**B1**

(12) # EUROPÄISCHE PATENTSCHRIFT

(45) Veröffentlichungstag der Patentschrift: **14.01.87**

(51) Int. Cl.⁴: **C 07 C 31/08**, C 07 C 29/32

(21) Anmeldenummer: **84109725.6**

(22) Anmeldetag: **16.08.84**

(54) **Verfahren zur Herstellung von Ethanol.**

(30) Priorität: **24.08.83 DE 3330507**

(43) Veröffentlichungstag der Anmeldung: **10.04.85 Patentblatt 85/15**

(45) Bekanntmachung des Hinweises auf die Patenterteilung: **14.01.87 Patentblatt 87/3**

(84) Benannte Vertragsstaaten: **DE FR GB IT NL SE**

(56) Entgegenhaltungen:
**EP-A-0 001 937**
**EP-A-0 010 373**
**EP-A-0 056 679**
**EP-A-0 109 645**
**WO-A-81/03327**
**DD-A-151 301**
**DE-A-3 228 820**
**GB-A-2 036 739**

(73) Patentinhaber: **Ruhrchemie Aktiengesellschaft, Bruchstrasse 219, D-4200 Oberhausen 13 (DE)**

(72) Erfinder: **Lipps, Wolfgang, Dr. Dipl.- Chem., Falkestrasse 67, D-4200 Oberhausen 11 (DE)**
Erfinder: **Bahrmann, Helmut, Dr. Dipl.- Chem., Rohstrasse 48, D-4236 Hamminkeln- Brünen (DE)**
Erfinder: **Cornils, Boy, Dr. Dipl.- Chem., Friedrich-Ebert- Strasse 45, D-4220 Dinslaken (DE)**
Erfinder: **Konkol, Werner, Dr. Dipl.- Chem., Lützowstrasse 40 a, D-4200 Oberhausen 11 (DE)**

(74) Vertreter: **Reichelt, Karl- Heinz, Dr., m. Br. Ruhrchemie Aktiengesellschaft Abt. PLD Postfach 13 01 60, D-4200 Oberhausen 13 (DE)**

EP 0 136 499 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein Verfahren zur Herstellung von Ethanol aus Methanol und Synthesegas, d.h. dem Gemisch von Kohlenmonoxid und Wasserstoff in Gegenwart von Kobalt und Rhodium als Katalysatoren. Diese Reaktion, sie wird als Homologisierung bezeichnet, ermöglicht es, ausgehend von Methanol durch Einführung einer oder mehrerer $CH_2$-Gruppen höhert homologe Alkohole herzustellen.

Die Homologisierung findet im verstärketn Maße Interesse. da sie einen Weg zur Gewinnung höherer Alkohole eröffnet, der unabhängig von der Verhendung von Erdöl ist. Als Einsatzstoff wird Synthesegas bzw. daraus hergestelltes Methanol benötigt; Synthesegas ist z.B. aus Kohle oder Erdgas nach verschiedenen. technisch bewährten Prozessen Zugänglich.

Es ist seit langem bekannt (vgl. z.B. DE-PS 86T 849). Methanol mit Wasserstoff und Kohlenaonoxid in Gegenwart eines wasserlöslichen Kobaltkatalysators bei hohen Temperaturen und Drücken in Ethanol umzuvandeln. Die Reaktion verl-uft nach folgender Summengleichung:

$CH_3OH + CO + 2H_2 \rightleftarrows C_2H_5OH + H_2O$

wobei in untergeordnetem Maße höhere Alkohole entsprechend

$CH_3OH + n(CO + 2H_2) \rightleftarrows CH_3(CH_2)_nOH + n\ H_2O$

gebildet werden können.

Während ursprünglich für die Reaktzon ausschließlich Kobalt als Katalysator verwendet wurde gewannen im Laufe der Zeit Mehrkoaponenten-Katalysatoren Zunehmend Bedeutung.

In der DE-0S 30 I6 7I5 ist die Herstellung von Ethanol aus Methanol unter Verwendung von Kobaltsulfid als Katalysator, der eine Stickstoff enthaltende Verbindung und/oder eine Phosphorverbindung aufweist beschrieben. Als stickstoffhaltige Verbindung werden Amide, Natrile, Trialkylamine und heterocyclische Verbindungen eingesetzt. Die beanspruchten Maßnahmen sollen sich vorteilhaft auf die Selektivität der Reaktion von Methanol zu Ethanol auswirken.

Das gleiche Ziel wird nach dem Verfahren der GB-PS 20 36 739 mit einem Katalysatorsystem angestrebt das aus Kobalt Ruthenium oder Osmium Halogen und einer organischen Verbindung eines Elementes der Gruppe VA des Periodensystems der Elemente besteht

Nach der Lehre der US-PS 41 S3 966 gewinnt man Ethanol aus Methanol und Synthesegas unter Verwendung eines aus Kobaltacetylacetonat einer organischen Verbindung eines Elementes der Gruppe VA des Periodensysteas der Elemente, einer Rutheniumverbindung und einer Jodverbindung bestehenden Katalysators.

Über eine vollständige Inhibierung der Aktivität von Kobaltkatalysatoren bei der Hoaologisierungsreaktion, hervorgerufen durch die Anwesenheit Stickstoff enthaltender basischer Verbindungen wie $NH_4OH$ und Pyridin berichtet J. Berty in Chen. Techn. B (1956) 260. In der jüngeren Literatur wird dieser inhibierende Effekt von H.Beuther et al. in "Epthyl fuel from nonpetroleua sources". Am. Chem.Soc. I79th meeting, Houston 23-29.3.1980 bestätigt.

Trotz der vorstehend beschriebenen Maßnahmen reicht die erzielte Selektivltät der Reaktion für eine technische Anwendung nicht aus. Hierbei ist zu berücksichtigen. daß die Nebenprodukte nicht nur in großer Menge, sondern in Form zahlreicher, unterschiedlicher Einzelverbindungen anfallen. So werden neben den erwünschten Alkoholen z.B. Methan, Ethan, Propan, verschiedene Ether sowie Methylacetat, Ethylacetat, Propylacetat, Acetaldehyd-damethylacetal, Acetaldehydmethylethylacetal und Acetaldehyddiethylacetal gebildet. Bei industrieller Nutzung des Prozesses ist daher ein hoher Aufwand erforderlich, um die aus den Nebenprodukten gewinnbaren Wertproduktanteile, z.B. durch Hydrierung, Verseifung und Destillation zu isolieren.

Eine Verbesserung der Selektavität der Umsetzung durch Zusatz eines Lösungsmittels zu den Reaktzonspartnern hat eine erhebliche Abnahme des Umsatzes, bezogen auf Reaktorvolumen und Zeit, zur Folge.

Nach den bekannten Verfahren kann man also entweder Methanol mit zufriedenstellender Selektivität in höhere Alkohole überführen, erzielt dann aber nur geringe Umsätze, oder man erreicht hohe Umsätze bei niedriger Selektivität.

Ferner erweisen sich viele Verbindungen von Elementen der Gruppe VA des Periodensystems der Elemente, die als Promotoren dem Kobaltkatalysator beigegeben werden, unter den extremen Versuchsbedingungen der Homologisierungsreaktion als wenig stabil und werden im Laufe der Reaktion in andere Komponenten umgewandelt, deren katalytische Wirksamkeit nicht mehr gegeben ist. So werden z.B. dreibindige Phosphorverbindungen (Phosphine) im Laufe der Reaktion vollständig in eine Vielzahl von phosphorhaltigen Verbindungen zersetzt (vergleiche J.B. Keister und R. Gentile. J.Organomet.Chem.. 222 (1981) 143). Ähnliches Verhalten ist auch von Homologen des Phosphors, wie z.B. Aminen, Arsinen und Stibinen zu erwarten. Es ist daher, um einen Wiedereinsatz des Katalysators mit Hilfe eines Katalysatorkreislaufes sicherzustellen, notwendig, den Kobaltkatalysator mit unter Reaktionsbedingungen stabilen Promotoren zu versetzen, da nur mit solchen Verbindungen auch langzeitstabile Katalysatoren erhalten werden können.

Es bestand daher die Aufgabe, eine Arbeitsweise zu entwickeln, die es erlaubt, Methanol in Ethanol mit hoher Selektivität und hohem Umsatz zu überführen, sowie die Anzahl der Nebenprodukte wesentlich zu reduzieren, so daß das Reaktionsgemisch in einfacher Weise aufgetrennt werden kann.

Die Erfindung löst diese Aufgabe mittels eines Verfahrens zur Herstellung von Ethanol durch Umsetzung von Methanol mit Kohlenmonoxid und Wasserstoff, die im Volumverhältnis CO : $H_2$ gleich 1 : 1,0 bis 1 : 3,5

eingesetzt werden, bei Drucken von 100 bis 800 bar und Temperaturen von 180 bis 230°C in Gegenwart eines Kobalt/Jod-Katalysators, wobei das molare Verhältnis von Kobalt zu Methanol gleich 1 : 50 bis 1 : 5 000 beträgt, dadurch gekennzezchnet, daß der Katalysator zusätzlich eine Rhodiumverbindung und ein organisches Amin, Amid oder ein Harnstoffderivat enthält.

Kobalt wird dem Reaktionsgemisch im allgemeinen in Form eines Salzes wie Kobalt-2-ethylhexanoat, Kobaltacetylacetonat, Kobalthalogenid, Kobaltnitrat, als Oxid oder Hydroxid zugesetzt. Besonders bewährt hat sich Kobaltcarbonat. Es ist aber auch möglich, metallisches Kobalt in feinverteilter Form zu verwenden. Unter den Reaktionsbedingungen wandelt sich Kobalt bzw. die Kobaltverbindung mit Kohlenmonoxid und Wasserstoff unter Bildung eines Kobalt-Carbonyls oder -Hydrocarbonyls um.

Ein weiterer Bestandteil des Katalysatorsystems ist Rhodium, das dem Reaktionsgemisch als Verbindung, die sich unter den Reaktionsbedingungen im Reaktionsmedium löst, z.B. als Rhodium-halogenid, Rhodium-2-ethylhexanoat, Rhodium-acetylacetonat, Rhodium-acetat zugesetzt wird.

Wesentlich für das erfindungsgemäße Verfahren ist neben Rhodium die Gegenwart von organischen Aminen, Amiden oder Harnstoffderivaten.

Als organisches Amin verwendet man eines, das mindestens eine, gegebenenfalls kernsubstituierte Arylgruppe enthält.

Das organische Amid soll die allgemeine Formel

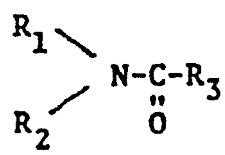

aufweisen, wobei $R_1$, $R_2$ und $R_3$ gleich oder verschieden sind und für geradkettige und/oder verzweigte Alkylreste mit 1 bis 16 Kohlenstoffatomen und/oder Arylreste mit 6 bis 15 Kohlenstoffatomen stehen, und die Reste $R_1$ und $R_3$ gegebenenfalls miteinander verbunden sind.

Das Harnstoffderivat soll der allgemeinen Formel

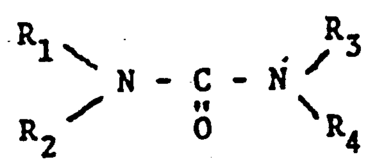

entsprechen. wobei $R_1$, $R_2$, $R_3$ und $R_4$ gleich oder verschieden sind und für Wasserstoff, geradkettige und/oder vereweigte Alkylreste mit jeweils 1 bis 16 Kohlenstoffatomen oder Arylreste mit 6 bis 15 Kohlenstoffatomen stehen und die Reste $R_1$ und $R_3$ gegebenenfalls miteihander verbunden sind.

Es ist überraschend, daß die Zugabe der Stickstoff enthaltenden Verbindungen nicht, wie aus der Literatur durch J. Berty und H. Beuther bekannt, zu einer Inhibierung des Katalysatorsystems führt. Vielmehr ist festzustellen, daß sich die Verwendung von organaschen Aminen, Amiden bzw. Harnstoffderavaten vorteilhaft auf die Standzeit des Katalysatorsystems und auf die Selektivität der Reaktion auswirkt.

Mit besonderem Vorteil werden folgende Amine, Amide und Harnstoffderivate in die Reaktion eingesetzt: N-Methylpyrrolidon-2, Triphenylamin, Dimethylacetamid, Tetramethylharnstoff, Propylenhanstoff (Perhydropyrimidin-2-on), Succinimid, 2-Chloranilin, 4-Chloranilin, Diphenylamin, N-Benzyl-2-pyrrolidon, N-Benzylpyridinium-3-sulfonat, 1,3-Dimethylimidazolidin-2-on oder N.N'-Diphenylharnstoff.

>Das im erfindungsgemäßen Verfahren eingesetzte Katalysatorsystem kann man dem Reaktionsgemisch in Form seiner einzelnen Bestandteile zuführen. Eine Präformierung der Metallkomplexverbindungen, die Komponenten des Katalysatorsystems sind, ist nicht erforderlich. Das Katalysatorsystem ist wiederholt einsetzbar.

Die Carbonyle bildenden Katalysatoren bzw. Katalysatorkomponenten können in einem hochsiedenden Lösungsmittel wie Diethylenglykoldimethylether, Tetraethylenglykoldimethylether, Neopentylglykol (2.2-Dimethyl-1.3-propan-diol), Ethylenglykol oder Trikresylphosphat suspendiert bzw. aufgelöst werden. Besonders geeignet als Lösungs- bzw. Suspensionsmittel sind die höher als Ethanol bzw. n-Propanol siedenden organischen Nebenprodukte der Reaktion.

Das als Ausgangsstoff eingesetzte Methanol kann in Form des in technischen Anlagen hergestellten Produktes mit einem Wassergehalt von 4 - 6 % verwendet werden. Eine zusätzliche Reinigung ist nicht erforderlich.

Das ursprunglich eingesetzte Reaktionsgemisch enthält bis zu 25 Gew.% Wasser. bezogen auf Methanol. Höhere Wassermengen beeinflussen den Umsatz nur unwesentlich. Zweckmäßig wird das Wasser zusammen mit dem Methanol dem Reaktor zugeführt.

Das molare Verhältnis von Kobalt zu Methanol beträgt 1 : 30 bis 1 : 5 000 insbesondere 1 : 30 bis 1 : 2 000. Kobalt und organisches Amin. Amid bzw. Harnstoffderivat werden in einem molaren Verhältnis von 1 : 0.5 bis 1: 1 000 vorzugsweise 1 : 1 bis 1 : 50 eingesetzt.

Das molare Verhältnis von Rhodium zu Kobalt beträgt 0.001 : 1 bis 2 : 1 vorzugsweise 0.01 : 1 bis 0.3 : 1.

Kobalt und Jod bzw. Jodid werden im molaren Verhältnis von 1 : 0.02 bis 1 : 2 insbesondere 1 : 0.05 bis 1 : 1 angewandt.

Das Kohlenmonoxid-/Wasserstoff-Gemisch kann Verunreinigungen wie Schwefel enthalten. Die Aktivität wird dadurch nicht negativ beeinflußt. Kohlendioxid und/oder Stickstoff bis zu 4 Vol.-%. bezogen auf das Gesamtgemisch, schaden nicht.

Der neue Prozeß kann sowohl diskontinuierlich als auch kontinuierlich durchpeführt werden. Im allgemeinen erfolgt die Umsetzung von Methanol. Kohlenmonoxid und Wasserstoff bei Temperaturen von 150 bis 250°C. insbesondere 180 bis 230°C. Der Druck wird auf Werte zwischen 100 bis 800 bar gehalten. Das molare Verhältnis von Wasserstoff zu Kohlenmonoxid im Synthesegas beträgt 1 : 1 bis 3.5 : 1.

Die nachstehend beschriebenen Beispiele erläutern die Erfindung.

**Beispiel 1**

In einem Stahlautoklaven (1 l Inhalt), versehen mit Rührer, Temperaturmessung, Probenahmestutzen und einem Gasometer, der zum Auffangen gasförmiger Bestandteile dient, werden 300 g (9375 mmol) Methanol, 3 g (25,22 mmol) $CoCO_3$, 260 mg (0,00253 g-atom) Rh (entsprechend 1239 mg Rh(III)-2-ethylhexanoat), 4,16 g (27,75 mmol) NaJ sowie 5,5 g (55,48 mmol) N-Methylpyrrolidon-2 vorgelegt, Anschließend wird mit Synthesegas (CO : $H_2$ - 1 : 2) ein Druck von 550 bar eingestellt, auf 200°C erhitzt und die Reaktion 2 Stunden lang unter Nachpressen von Synthesegas fortgesetzt. Nach Abkühlen des Reaktionsgemisches wird in den Gasometer entspannt. Die erzielten Umsätze und Selektivztäten der Beispiele 1 bis 11 sind in Tabelle 1 angegeben. Sie beruhen auf gaschromatographischen Analysen des Reaktionsproduktes.

Rhodium wird in allen Beispielen, in denen mit Rhodium gearbeitet wird, als Rh-2-ethylhexanoat eingesetzt. aber gerechnet als Metall angegeben.

**Beispiele 2 - 4**

Diese Versuche werden analog Beispiel 1 durchgeführt, abweichend davon werden anstatt 260 mg Rh in jedem der Beispiele 300 mg (0,00292 g-atom) Rh eingesetzt und anstatt 5,5 g (55,48 mmol) N-Methylpyrrolidon-(2) werden in Versuch 2: 13,75 g (138,7 mmol) N-Methylpyrrolidon-(2) in Versuch 3 : 27,5 g (277,4 mmol) N-Methvlpyrrolidon-(2), und in Versuch 4: 45,6 g (460 mmol) N-Methvlpyrrolidon-(2) zugesetzt.

**Beispiel 5 - 7**

Die Versuchsdurchführung erfolgt analog Beispiel 2, abweichend davon werden anstatt 300mg Rh in Versuch 5: 130 mg (0,00126 g-atom) Rhodium in Versuch 6: 260 mg (0,00253 g-atom) Rhodium und in Versuch 7: 520 mg (0,0051 g-atom) Rhodium zugesetzt.

Die Versuchsergebnisse sind in Tabelle 1 zusammengestellt.

**Beispiel 8**

Ein Stahlautoklav von 2 l Inhalt, versehen mit Rührer, Probenahmestutzen, Temperaturmessung und einem Gasometer zum Auffangen gasförmiger Bestandteile, wird mit 600 g (18750 mmol) Methanol, 6 g (50,44 mmol) $CoCO_3$, 520 mg (0 0051 g-atom) Rh, 8,32 g (55,5 mmol) NaJ sowie 68 g (277,18 mmol) Triphenylamin beschickt. Nach 2 Stunden Reaktxonszeit bei 200°C sowie einem Druck von 550 bar (Synthesegas CO : $H_2$- 1 : 2) wird abgekühlt und in den Gasometer entspannt. Die Versuchsergebnisse sind in Tabelle 1 zusammengestellt.

**Beispiel 9**

Unter den in Beispiel 8 beschriebenen Reaktionsbedingungen werden 600 g (18750 mmol) Methanol, 6 g (50,44 mmol) $CoCO_3$, 300 mg (0,00292 g-atom) Rh, 3,8 g (25,35 mmol) NaJ sowie 45,6 g (460 mmol) N-Methylpyrrolidon-2 umgesetzt.

**Beispiel 10**

300 g (9375 mmol) Methanol werden mit Synthesegas ($CO/H_2$) in Gegenwart von 3 g (25,22 mmol) $CoCO_3$, 300 mg (0,00292 g-atom) Rh, 3,8 g (25,35 mmol) NaJ und 45,6 g (460 mmol) N-Methylpyrrolidon-2 als Katalysator gemäß Beispiel 1 umgesetzt. Die Versuchsergebnisse sind in Tabelle 1 zusammengestellt.

**Beispiel 11**

600 g (18750 mmol) Methanol werden mit Synthesegas in Gegenwart von 6 g (50,44 mmol) $CoCO_3$, 520 mg (0,0051 g-atom) Rh, 8,32 g (55,5 mmol) NaJ sowie 16,2 g (139,5 mmol) Tetramethylharnstoff als Katalysator 4 Stunden unter den in Beispiel 8 beschriebenen Reaktionsbedingungen umgesetzt.

Die Versuchsergebnisse sind in Tabelle 1 zusammengestellt.

**Beispiel 12**

300 g (9375 mmol) Methanol werden mit Synthesegas in Gegenwart von 3 g (25.22 mmol) $CoCO_3$, 4,16 g (27,75 mmol) NaJ, 260 mg (0,00253 g-atom) Rhodium sowie 3,22 g (27,72 mmol) Tetramethylharnstoff als Katalysator gemäß Beispiel 1 zur Reaktion gebracht.

Die Versuchsergebnisse sind in Tabelle 2 zusammengestellt.

**Beispiel 13-15**

Die Versuche der Beispiele 13-15 werden analog Beispiel 12 durchgeführt, in Beispiel 13 werden abweichend davon 130 mg (0,00126 g-atom) Rh, 3,95 g (26,35 mmol) NaJ sowie 6,12 g (52,69 mmol) Tetramethylharnstoff, in Beispiel 14 wird wie Beispiel 12 gearbeitet, aber 16,11 g (138,7 mmol) Tetramethylharnstoff zugegeben, in Beispiel 15 wird wie in Beispiel 12 gearbeitet, aber 6,45 g (55,53 mmol) Tetramethylharnstoff eingesetzt.

Die Versuchsergebnisse sind in Tabelle 2 zusammengestellt.

**Beispiel 16**

600 g (18750 mmol) Methanol werden mit Synthesegas in Gegenwart von 6 g (50,44 mmol) $CoCO_3$, 520 mg (0,0051 g-atom) Rhodium, 8,3 g (55,37 mmol) Natriumjodid sowie 27,8 g (277,7 mmol) N,N'-Propylenharnstoff (Perhydro-pyrimidin-2-on) als Katalysator gemäß Beispiel 1 umgesetzt.

Die Versuchsergebnisse sind in Tabelle 2 zusammengestellt.

**Beispiel 17**

Es wird wie in Beispiel 16 angegeben gearbeitet, jedoch werden anstelle des in Beispiel 16 eingesetzten 27,8 g (277,7 mmol) N,N'-Propylenharnstoffs (Perhydro-pyrinidin-2-on) 13,8 g (139,27 mmol) Succinimid eingesetzt.

Die Versuchsergebnisse sind in Tabelle 2 zusammengestellt.

**Beispiel 18**

300 g (9375 mmol) Methanol werden mit Synthesegas in Gegenwart von 3 g (25,22 mmol) $CoCO_3$, 260 mg (0,00253 g-atom) Rh, 4,16 g (27,75 mmol) Natriumjodid sowie 7,1 g (55,66 mmol) 2-Chloranilin als Katalysator gemäß Beispiel 1 umgesetzt.

Die Versuchsergebnisse sind in Tabelle 2 zusammengestellt.

**Beispiel 19**

Anstelle der in Beispiel 18 eingesetzten N-organischen Komponente 2°Chloranilin wird mit der gleichen Menge 4-Chloranilin gearbeitet.
Die Versuchsergebnisse sind in Tabelle 2 zusammengestellt.

**Beispiel 20**

300 g (9375 mmol) Methanol werden mit Synthesegas in Gegenwart von 3 g (25,22 mmol) $CoCO_3$, 260 mg (0,00253 g-atom) Rh, 4,16 g (27,75 mmol) NaJ sowie 9,4 g (55,55 mmol) Diphenylamin als Katalysator gemäß Beispiel 1 umgesetzt.
Die Versuchsergebnisse sind in Tabelle 2 zusammengestellt.

**Beispiel 21**

300 g (9375 mmol) Methanol werden mit Synthesegas in Gegenwart von 3 g (25,22 mmol) $CoCO_3$, 260 mg (0,00253 g-atom) Rhodium, 4,16 g (27,75 mmol) NaJ und 9,72 g (55,64 mmol) N-Benzyl-2-pyrrolidon als Katalysator gemäß Beispiel 1 umgesetzt.
Die Versuchsergebnisse sind in Tabelle 2 zusammengestellt.

**Beispiel 22**

Es wird wie in Beispiel 21 angegeben gearbeitet, jedoch werden anstelle von N-BenzylPyrrolidon-(2) 13,8 g (55,36 mmol) N-Benzylpyridinium-3-sulfonat eingesetzt. Die Versuchsergebnisse sind in Tabelle 2 zusammengestellt.

**Beispiel 23**

300 g (9375 mmol) Methanol werden mit Synthesegas in Gegenwart von 3 g (25,22 mmol) $CoCO_3$, 260 mg (0,00253 g-atom) Rhodium, 4,16 g (27,75 mmol) NaJ sowie 6,33 g (55,45 mmol) 1.3-Dimethylimidazolidin-2-on als Katalysator gemäß Beispiel 1 umgesetzt.
Die Versuchsergebnisse sind in Tabelle 3 zusammengestellt.

**Beispiel 24**

Es wird wie in Beispiel 21 angegeben gearbeitet, jedoch werden anstelle von N-Benzylpyrrolidon-(2) 11,78 g (55,5 mmol) N.N'-Diphenylharnstoff eingesetzt.
Die Versuchsergebnisse sind in Tabelle 3 zusammengestellt.

**Beispiel 25** (Vergleichsbeispiel)

300 g (9375 mmol) Methanol werden mit Synthesegas in Gegenwart eines nur aus 3 g (25,22 mmol) $CoCO_3$, und 3,8 g (25,35 mmol) NaJ bestehenden Katalysator gemäß Beispiel 1 umgesetzt.
Auf einen Zusatz von Rhodium und organischen Amins. Amids bzw. Harnstoffdervats wird verzichtet.
Die Versuchsergebnisse sind in Tabelle 3 zusammengestellt.

**Vergleichsbeispiel 26**

Ein Katalysatorsystem gemäß der DE-OS 30 16 715, bestehend aus 20 g (217,4 mmol) Kobaltsulfid, 40 g (98,85 mmol) Tributylphosphin sowie 100 g (1008,8 mmol) N-Methylpyrro-lidon-(2) wird mit 200 g (6242 mmol) Methanol vermischt und bei 200 bar und 200°C mit Synthesegas ($CO:H_2$, = 1:2) in dem in Beispiel 1

beschriebenen Autoklaven behandelt (vgl. Beispiel 26 a in Tabelle 3). Die in Tabelle 3 zusammengestellten Versuchsergenisse zeigen, daß die in der obigen Schrift angegeleanen Umsätze und Selektivitäten (vgl. Beispiel 26 b in Tabelle 3) nicht erzielt werden.

Wegen der sehr unspezifischen Produktverteilung sowie des niedrigen Umsatzes empfiehlt sich, wie der Vergleich mit den Beispielen 1 - 24 zeigt, eine technische Anwendung dieses Katalysatorsystems nicht.

**Tabelle 1:** Versuchsergebnisse der Beispiele 1 - 11

| Beispiel | 1 | 2 | 3 | 4 | 5 | 6 | 7 | 8 | 9 | 10 | 11 |
|---|---|---|---|---|---|---|---|---|---|---|---|
| Umsatz (Mol.-%) | 32 | 32 | 28 | 40 | 33 | 30 | 32 | 58 | 55 | 38 | 49 |
| Selektivitäten (Mol.-%) | | | | | | | | | | | |
| Ethanol | 62 | 64 | 67 | 67 | 58 | 63 | 58 | 63 | 61 | 68 | 62 |
| n-Propanol | 4 | 3 | 5 | 3 | 4 | 3 | 4 | 5 | 3 | 3 | 3 |
| Kohlenwasserstoffe | 13 | 14 | 7 | 11 | 14 | 14 | 14 | 8 | 10 | 11 | 10 |
| Acetaldehyd | 1 | 1 | 1 | 1 | 1 | 1 | 1 | 2 | 1 | - | 1 |
| Ether | 5 | 6 | 8 | 8 | 5 | 6 | 5 | 7 | 9 | 8 | 9 |
| Ester | 2 | 3 | 3 | 4 | 3 | 3 | 3 | 3 | 6 | 4 | 5 |
| Acetale | 7 | 3 | 2 | 1 | 11 | 3 | 11 | 8 | 4 | 1 | 3 |
| Höhere Alkohole | 3 | 4 | 4 | 4 | 3 | 4 | 3 | 2 | 5 | 4 | 7 |
| Rest | 3 | 3 | 3 | 1 | 2 | 3 | 2 | 2 | 1 | 1 | 0 |

| Beispiel | 12 | 13 | 14 | 15 | 16 | 17 | 18 | 19 | 20 | 21 | 22 |
|---|---|---|---|---|---|---|---|---|---|---|---|
| Umsatz (Mol.-%) | 66 | 43 | 26 | 48 | 34 | 17 | 38 | 45 | 59 | 55 | 35 |
| Selektivität (Mol.-%) | | | | | | | | | | | |
| Ethanol | 63 | 70 | 66 | 65 | 75 | 64 | 44 | 46 | 60 | 51 | 53 |
| n-Propanol | 4 | 4 | 4 | 4 | 4 | 4 | 5 | 4 | 4 | 2 | 1 |
| Kohlenwasserstoffe | 6 | 8 | 8 | 8 | 3 | 7 | 6 | 6 | 7 | 4 | 6 |
| Acetaldehyd | 1 | <0,1 | 1 | <0,1 | <0,1 | 2 | 1 | <0,1 | <0,1 | 1 | 1 |
| Ether | 6 | 7 | 8 | 8 | 5 | 8 | 7 | 3 | 6 | 6 | 6 |
| Ester | 5 | 3 | 5 | 3 | 5 | 6 | 8 | 9 | 6 | 10 | 10 |
| Acetale | 13 | 2 | 2 | 5 | 3 | 1 | 25 | 26 | 10 | 21 | 18 |
| Höhere Alkohole | 6 | 5 | 6 | 5 | 4 | 7 | 3 | 4 | 4 | 3 | 3 |
| Rest | 2 | 1 | - | 2 | 1 | 1 | 1 | 2 | 3 | 2 | 2 |

Tabelle 2: Versuchsergebnisse der Beispiele 12 - 22

| Beispiel | 23 | 24 | 25 | 26a | 26b |
|---|---|---|---|---|---|
| Umsatz (Mol.-%) | 78 | 42 | 64 | 15 | 32,5 |
| Selektivitäten (Mol-%) | | | | | |
| Ethanol | 59 | 59 | 19 | 37 | 90,1 |
| n-Propanol | 7 | 6 | 1 | 1 | +) |
| Kohlenwasserstoffe | 5 | 6 | 14 | 12 | +) |
| Acetaldehyd | < 0,1 | 1 | 7 | - | 2,4 |
| Ether | 6 | 8 | 7 | 18 | +) |
| Ester | 4 | 8 | 8 | 21 | 3,1 |
| Acetale | 11 | 10 | 37 | 5 | +) |
| Höhere Alkohole | 6 | 1 | 3 | 2 | +) |
| Rest | 2 | 1 | 4 | 4 | +) |

+) keine Angaben in der DE-OS 30 16 715

Tabelle 3: Versuchsergebnisse der Beispiele 23-26b

**Patentansprüche**

1. Verfahren zur Herstellung von Ethanol durch Umsetzung von Methanol mit Kohlenmonoxid und Wasserstoff, die im Volumverhältnis CO : $H_2$ gleich 1 : 1,0 bis 1 ; 3,5 eingesetst werden, bei Drucken von 100 bis 800 bar und Temperaturen von 180 bis 230°C in Gegenwart eines Katalysators bestenend aus Kobalt, einer weiteren Verbindung eines Elementes der Gruppe VIII des Periodensystems der Elemente, Jod oder Jodid und einer trivalenten Verbindung eines Elementes der Gruppe Va des Periodensystems der Elemente, wobei das molare Verhältnis von Kobalt : Jod oder Jodid 1 : 0,02 bis 1 : 2, das von Kobalt zu Methanol = 1 : 30 bis 1 : 5000 beträgt, dadurch gekennzeichnet, daß die weitere Verbindung eines Elementes der Gruppe VIII des Periodensystems der Elemente eine Rhodiumverbindung und die trivalente Verbindung ein organisches Amin und/oder Amid und/oder Harnstoffderivat ist.

2. Verfahren nach Anspruch 1, dadurch gekennzeichnet, daß das organische Amin mindestens eine, gegebnenfalls kernsubstituierte Arylgruppe enthält.

3. Verfahren nach Anspruch 1, dadurch gekennzeichnet, daß das organische Amid die allgemeine Formel

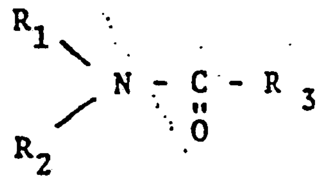

aufweist, wobei $R_1$, $R_2$ und $R_3$ gleich oder verschieden sind und für geradkettige und/oder verzweigte Alkylreste mit jeweils 1 bis 16 Kohlenstoffatomen und/oder Arylreste mit 6 bis 15 Kohlenstoffatomen stehen, und die Reste $R_1$ und $R_3$ gegebenenfalls miteinander verbunden sind.

4. Verfahren nach Anspruch 1, dadurch gekennzeichnet, daß das Harnstoffderivat die allgemeine Formel

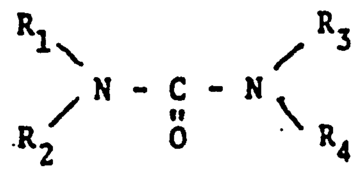

aufweist, wobei $R_1$, $R_2$, $R_3$ und $R_4$ gleich oder verschieden sind und für Wasserstoff, geradkettige und/oder verzweigte Alkylreste mit jeweils 1 bis 16 Kohlenstoffatomen und/oder Arylreste mit 6 bis 15 Kohlenstoffatomen stehen und die Reste $R_1$ und $R_3$ gegebenenfalls miteinander verbunden sind.

5. Verfahren nach Anspruch 1 bis 4, dadurch gekennzeichnet, daß das molare Verhältnis von Rhodium zu Kobalt 0.001 : 1 bis 2.0 : 1 und das molare Verhältnis von Kobalt zu organischem Amin, Amid bzw. Harnstoffderivat gleich 1 : 0.5 bis 1 : 1 000 beträgt.

6. Verfahren nach Anspruch 1 bis 5, dadurch gekennzeichnet, daß als organisches Amin, Amid bzw. Harnstoffderivat

N-Methylpyrrolidon-2
Triphenylamin
Dimethylacetamid
Tetramethylharnstoff
Propylenharnstoff (Perhydro-pyrimidin-2-on)
Succinimid
2-Chloranilin
4-Chloranilin
Diphenylamin
N-Benzyl-2-pyrrolidon
N-Benzylpyridinium-3-sulfonat
1.3-Dimethylimidazolidin-2-on oder
N.N-Diphenylharnstoff

eingesetzt werden.

## Claims

1. A process for the preparation of ethanol by the reaction of methanol with carbon monoxide and hydrogen which are employed in a volume ratio of CO : $H_2$ equalling 1 : 1.0 to 1 : 3.5 at pressures of 100 to 800 bar and temperatures of 180 to 230°C in the presence of a catalyst system consisting of cobalt, another compound of an element of the VIII Group of the Periodic System of elements, iodine or iodide and a trivalent compound of an element of the Va Group of the Periodic System of elements whereby the molar ratio of cobalt to iodine or iodide is 1 : 0.02 to 1 : 2, that of cobalt to methanol is 1 : 30 to 1 : 5000, characterised in that the other compound of the VIII Group of the Periodic System of elements is a rhoduim compound and the trivalent compound is an organic amine and/or amide and/or a urea derivative.

2. A process according to claim 1, characterised in that the organic amine contains at least one aryl group whose ring centre can be substituted.

3. A process according to claim 1, characterised in that the organic amide has the general formula

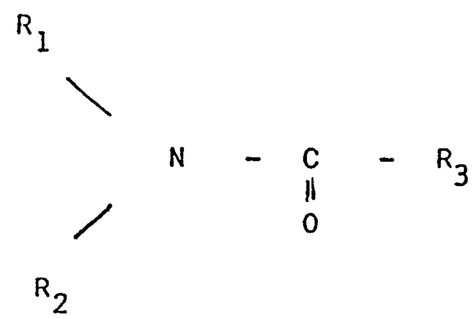

where $R_1$, $R_2$ and $R_3$ are the same or different and denote straight-chain and/or branched alkyl groups each with 1 to 16 carbon atoms and/or aryl groups with 6 to 15 carbon atoms and the groups $R^1$ and $R^2$ can be connected to each other.

4. A process according to claim 1, characterised in that the urea derivative has the general formula

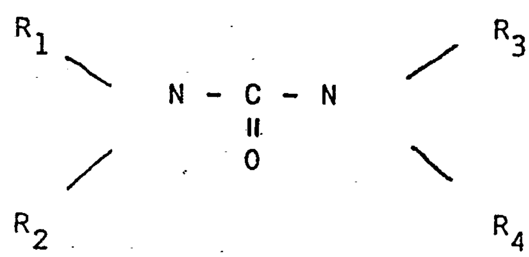

where $R_1$, $R_2$, $R_3$ and $R_4$ are the same or different and denote hydrogen, straight-chain and/or branched alkyl groups each with 6 to 16 carbon atoms and/or aryl groups with 6 to 15 carbon atoms and the groups $R_1$ and $R_3$ can be connected to each other.

5. A process according to claims 1 to 4, characterised in that the molar ratio of rhodium to cobalt is 0.001 : 1 to 2.0 : 1 and the molar ratio of cobalt to organic amine, amide or urea derivative is 1 : 0.5 to 1 : 1 000.

6. A process according to claims 1 to 5. characterised in that the following are employed as an organic amine, amide or urea derivative:

N-methylpyrrolidone-2
triphenylamine
dimethylacetamide
tetramethyl urea
propylene urea (perhydro-pyrimidine-2-one) succinimide
2-chloroaniline
4-chloroaniline
diphenylamine
N-benzyl-2-pyrrolidone
N-benzylpyridinium-3-sulfonate
1.3-diemthylimidazolidine-2-one or
N,N'-diphenyl urea.

**Revendications**

1. Procédé pour produire de l'ethanol par réaction du méthanol avec le monoxyde de carbone et l'hydrogène, que l'on utilise selon un rapport en volume CO : $H_2$ compris entre 1 : 1,0 et 1 : 3,5, sous des pressions de 100 à 800 bars et à des températures de 180 à 230°C en présence d'un catalyseur consistant en du cobalt, un autre composé d'un élément du groupe VIII du système périodique des éléments, l'iode ou un iodure et un composé trivalent d'un élément du groupe Va du système périodique des éléments, le rapport molaire du cobalt à l'iode ou à l'iodure se situant entre 1 : 0,02 et 1 : 2, le rapport molaire du cobalt au méthanol allant de 1 : 30 à 1 : 5 000, procédé caractérisé en ce que l'autre composé d'un élément du groupe VIII du système périodique des éléments est un composé de rhodium, et le composé trivalent est une amine organique et/ou un amide et/ou un dérivé d'urée organique.

2. Procédé selon la revendication 1, caractérisé en ce que l'amine organique contient au moins un groupe aryle éventuellement substitué sur le noyau.

3. Procédé selon la revendication 1, caractérisé en ce que l'amide organique présente la formule générale:

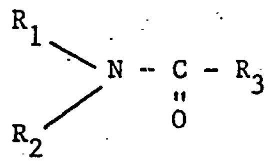

dans laquelle $R_1$, $R_2$ et $R_3$ sont identiques ou différents et représentent des restes alkyle linéaires et/ou ramifiés ayant chacun 1 à 16 atomes de carbone et/ou des restes aryle ayant 6 à 15 atomes de carbone, et les restes $R_1$ et $R_3$ sont éventuellement liés l'un à l'autre.

4. Procédé selon la revendication 1, caractérisé en ce que le dérivé d'urée présente la formule générale:

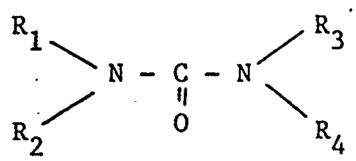

dans laquelle $R_1$, $R_2$, $R_3$ et $R_4$ sont identiques ou différents et représentent chacun un atome d'hydrogène, des restes alkyle linéaires et/ou ramifiés ayant chacun 1 à 16 atomes de carbone et/ou des restes aryle comportant 6 à 15 atomes de carbone, et les restes $R_1$ et $R_3$ sont éventuellement reliés l'un à l'autre.

5. Procédé selon la revendication 1 à 4, caractérisé en ce que le rapport molaire du rhodium au cobalt se situe entre 0,001 : 1 et 2,0 : 1 et en ce que le rapport molaire du cobalt à l'amine organique, l'amide ou le dérivé d'urée vaut de 1 : 0,5 à 1 : 1 000.

6. Procédé selon la revendication 1 à 5, caractérisé en ce qu'on utilise comme amine organique, amide ou dérivé d'urée:

la N-méthylpyrrolidone-2,
la triphénylamine,
le diméthylacétamide,
la tétraméthylurée,
la propylèneurée (perhydro-pyrimidine-2-one)
le succinimide,
la 2-chloraniline,
la 4-chloraniline,
la diphénylamine,
la N-benzyl-2-pyrrolidone,
le 3-sulfonate de N-benzylpyridinium,
la 1,3-diméthylimidazolidine-2-one, ou
la N,N'-diphénylurée.